Europäisches Patentamt

European Patent Office

Office européen des brevets

(11) Numéro de publication : **0 496 716 A1**

## (12) DEMANDE DE BREVET EUROPEEN

(21) Numéro de dépôt : **92870009.5**

(22) Date de dépôt : **13.01.92**

(51) Int. Cl.⁵ : **A61F 13/15**

(30) Priorité : **24.01.91 BE 9100059**

(43) Date de publication de la demande :
**29.07.92 Bulletin 92/31**

(84) Etats contractants désignés :
**AT BE CH DE DK ES FR GB GR IT LI LU MC NL PT SE**

(71) Demandeur : **Malak, Jean Clément Edouard Ghislain**
**2, rue de Versailles**
**B-7130 Binche (BE)**

(72) Inventeur : **Malak, Jean Clément Edouard Ghislain**
**2, rue de Versailles**
**B-7130 Binche (BE)**

(74) Mandataire : **Claeys, Pierre et al**
**Bureau Gevers rue de Livourne 7 Bte 1**
**B-1050 Bruxelles (BE)**

(54) **Couche-culotte, ou similaire, à jeter.**

(57) Bande à jeter (1) du type lange, couche, couche-culotte ou article analogue, comprenant un élément absorbant (5), une feuille imperméable aux fluides (2) dont une première face (4) supporte l'élément absorbant (5), une feuille étanche aux odeurs (7) qui est disposée à plat sur une deuxième face (3) de la feuille imperméable aux fluides (2) et qui comporte un bord périphérique dont une partie est fixée sur ladite deuxième face (3), le bord périphérique restant étant libre, ces deux feuilles formant ensemble une enveloppe en forme de poche avec une ouverture de poche (12) entre le bord périphérique resté libre de la feuille étanche aux odeurs (7) et la feuille imperméable aux fluides (2), et des moyens de fermeture capables de sceller l'ouverture de poche (12) d'une manière étanche aux odeurs.

*Fig.2*

EP 0 496 716 A1

La présente invention est relative à une bande à jeter du type lange, couche, couche-culotte ou article analogue, comprenant

un élément absorbant,

une feuille imperméable aux fluides dont une première face supporte l'élément absorbant,

une enveloppe en forme de poche dans laquelle l'élément absorbant peut être enfermé après usage, et

des moyens de fermeture de l'enveloppe.

On connaît depuis longtemps des bandes à jeter, destinées aux soins des bébés, du type langes, couches, couches-culottes ainsi qu'une série d'articles analogues pour incontinents ou grabataires. Actuellement, toutes ces bandes comportent un élément absorbant pour absorber des fluides et déchets corporels du porteur et une feuille imperméable empêchant les fluides absorbés de progresser à l'extérieur de la bande.

Les fluides et déchets corporels présentent l'inconvénient de dégager des odeurs désagréables. Habituellement les bandes sont jetées après usage. Toutefois, les bandes jetées peuvent parfois rester une journée entière ou davantage dans la poubelle d'une salle de bains par exemple, avant d'être évacuées. En effet, il est normal d'attendre que le sac, dont la poubelle est garnie, soit rempli pour l'évacuer. Pendant toute cette période, les odeurs dégagées par les bandes à jeter se propagent dans la salle où se trouve la poubelle, ce qui est très désagréable.

Dans d'autres circonstances, par exemple lors de voyages avec des enfants en bas âge, il peut être nécessaire de les langer alors qu'on se trouve dans un véhicule. Dans ces cas également, les odeurs dégagées par les bandes en question sont parfois difficiles à éviter, et surtout à supporter.

Il est par ailleurs connu de pourvoir des bandes d'hygiène féminine d'enveloppes dans lesquelles les bandes peuvent être enfermées après usage. Ces enveloppes peuvent être formées de pochettes de formes complexes, qui sont repliées sur elles-mêmes et fixées au dos des bandes hygiéniques (voir US -A-4692162, US-A-4182336 et US-A-4605403). On connaît également des couches munies de telles pochettes (voir US-A-4923455). Il est également connu de prévoir fixée au dos de bandes hygiéniques une large feuille imperméable qui est repliée sur elle-même et qui peut être dépliée puis repliée d'une autre manière autour de la bande usagée, afin d'envelopper celle-ci complètement (US-A-4857066).

Tous ces dispositifs connus présentent l'inconvénient commun de prévoir au dos de la bande à jeter, un système compliqué, qui augmente énormément le coût de fabrication de cet article et qui nécessite une succession de manipulations peu aisées pour finalement envelopper l'article à jeter d'une manière appropriée. Ils rendent l'article excessivement volumineux, ce qui doit gêner les porteurs.

La présente invention a pour but de remédier à ces problèmes par la mise au point d'une bande à jeter qui, après usage, empêche le dégagement des odeurs issues des fluides et déchets corporels contenus dans ou portés par l'élément absorbant de la bande. La bande à jeter suivant l'invention sera simple à fabriquer et ne demandera ni investissements coûteux ni appareillages complexes pour sa fabrication. Elle ne sera pas gênante à porter par l'utilisateur.

Avantageusement, la bande à jeter fournira en supplément un moyen de nettoyage permettant d'effectuer la toilette du porteur avant l'application d'une nouvelle bande.

Suivant l'invention, on résout les problèmes posés ci-dessus par une bande à jeter du type décrit au début, comprenant en outre

une feuille étanche aux odeurs qui est disposée à plat sur une deuxième face, opposée à la première, de la feuille imperméable aux fluides et qui comporte un bord périphérique dont une partie est fixée sur ladite deuxième face, le bord périphérique restant étant libre, ces deux feuilles formant ensemble ladite enveloppe en forme de poche avec une ouverture de poche entre le bord périphérique resté libre de la feuille étanche aux odeurs et la feuille imperméable aux fluides, et lesdits moyens de fermeture étant capables de sceller l'ouverture de poche d'une manière étanche aux odeurs.

Suivant une forme perfectionnée de réalisation de l'invention, en position d'utilisation de la bande, la feuille étanche aux odeurs présente une première face adjacente à la feuille imperméable aux fluides et une deuxième face, opposée à la première et tournée vers l'extérieur de la bande, et, en position de fermeture de l'enveloppe, la feuille étanche aux odeurs présente au moins partiellement sa deuxième face tournée vers l'intérieur de l'enveloppe et sa première face tournée vers l'extérieur de l'enveloppe. Avantageusement, l'ouverture de poche a une dimension suffisante pour permettre l'introduction d'une main à l'intérieur de la poche et un retournement de la feuille étanche aux odeurs autour de l'élément absorbant sans que la main soit souillée par l'élément absorbant contenant des résidus organiques.

D'autres détails et particularités de l'invention ressortiront de la description donnée ci-après, à titre non limitatif et avec référence aux dessins annexés.

La figure 1 représente une vue en plan d'une couche-culotte suivant l'invention.

Les figures 2 et 3 représentent des vues de cette couche-culotte pendant l'introduction de l'élément absorbant dans la poche.

La figure 4 représente une vue de la couche-culotte après fermeture de la poche.

Sur les différents dessins, les éléments identiques ou analogues sont désignés par les mêmes références.

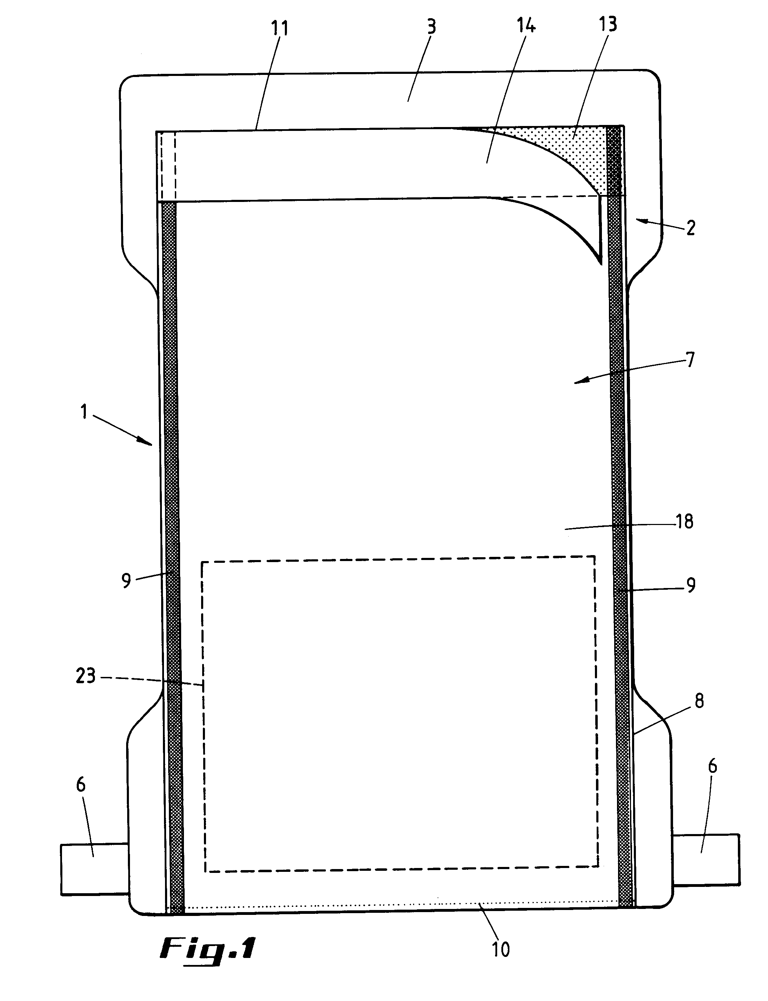

On peut voir sur la figure 1 un exemple de réalisation de couche-culotte 1 de l'extérieur, c'est-à-dire du côté non en contact avec la peau du porteur.

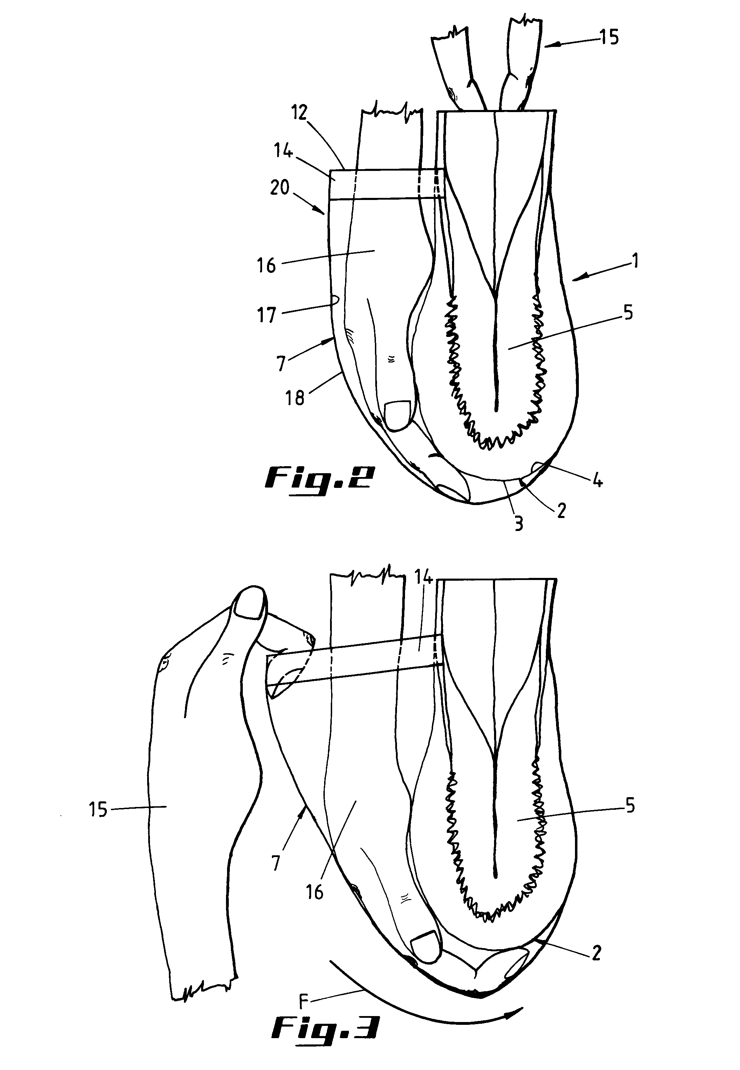

Cette couche-culotte 1 comprend une feuille imperméable aux fluides 2 usuelle qui présente deux faces, une face extérieure 3, et une face intérieure 4 (voir figure 2). Cette dernière est tournée vers le corps du porteur, en position d'usage de la couche-culotte 1, et elle supporte un élément absorbant 5 de type tampon, connu en soi. L'élément absorbant 5 et la feuille imperméable aux fluides 2 sont d'une manière connue fixés l'un à l'autre. Pour fixer la couche-culotte au corps du porteur, des pattes d'attache 6, à une face adhésive, sont prévues, d'une manière connue en soi, à une extrémité de la couche-culotte.

Dans l'exemple de réalisation illustré sur les figures 1 à 4, la couche-culotte 1 comprend en outre une feuille étanche aux odeurs 7, qui est disposée à plat sur la face 3 de la feuille imperméable aux fluides 2. Cette feuille peut par exemple être en une matière plastique, et particulièrement avantageusement en une matière identique à celle formant la feuille imperméable aux fluides 2 usuelle. Elle pourrait bien sûr aussi être dans une autre matière, comme par exemple dans certaines qualités de papier imperméabilisé.

La feuille étanche aux odeurs 7 a, dans cet exemple de réalisation, une forme sensiblement rectangulaire un peu plus petite que la feuille imperméable aux fluides 2. Elle présente donc 4 côtés formant un bord périphérique 8. Celui-ci est, sur trois de ses côtés, fixé sur la face 3 de la feuille imperméable aux fluides, par tout moyen approprié. Pour cette fixation, on peut par exemple imaginer un collage, un thermosoudage, ou tout autre processus analogue. Sur la figure 1, les deux côtés longitudinaux du bord périphérique 8 de la feuille 7 sont collés sur la feuille 2 le long de deux bandes de colle 9. Un des deux côtés transversaux est par ailleurs représenté soudé le long d'une ligne de thermosoudage 10.

Le côté restant du bord périphérique 8, c'est-à-dire le côté 11, reste libre. Ainsi, les deux feuilles 2 et 7 forment ensemble une enveloppe en forme de poche 20, du type poche de kangourou, avec une ouverture de poche 12 (voir figure 2).

La couche-culotte suivant l'invention comprend en outre des moyens de fermeture de la poche ainsi formée, et donc en particulier de son ouverture 12, de façon à la rendre étanche aux odeurs. Dans l'exemple de réalisation illustré sur les dessins ces moyens de fermeture comprennent un ruban adhésif double face 13 connu en soi. L'une de ses faces est déjà collée le long du côté II du bord périphérique 8 de la feuille étanche aux odeurs 7. L'autre de ses faces est recouverte d'une languette de protection 14 détachable qui, sur la figure 1, est représentée partiellement détachée. Il est évident que d'autres moyens de fermeture pourraient être prévus. Par exemple on pourrait utiliser des bandes autoagrippantes de type Velcro. On pourrait aussi agencer plutôt le ruban adhésif sur la feuille imperméable aux fluides 3.

Après utilisation de la couche-culotte qui vient d'être décrite, on détache celle-ci du porteur et, comne d'habitude, on replie en deux la couche-culotte 1 comme représenté sur la figure 2. Il est alors possible de retenir entre deux doigts d'une main 15 les extrémités de la couche-culotte 1 repliée et d'introduire l'autre main 16 par l'ouverture de poche 12 à l'intérieur de la poche formée (voir figure 2).

Lorsque, ainsi qu'il est illustré sur la figure 3, la main 16 a pénétré suffisamment profondément dans la poche, sans aucun risque d'être souillée, il est possible de maintenir la couche-culotte 1 dans son état replié par cette main-là 16. L'autre main 15 est alors utilisée pour retourner la feuille étanche aux odeurs 7 autour de la couche-culotte 1, dans le sens de la flèche F. La manipulation est simple, rapide et propre.

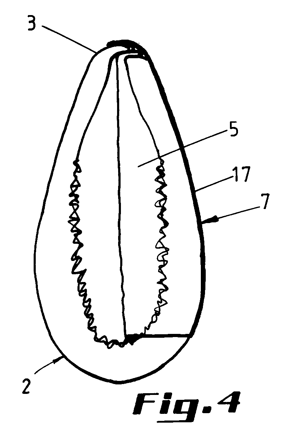

En position d'utilisation ou de stockage de la couche-culotte 1 suivant l'invention, la feuille 7 présente une première face 17 adjacente à la feuille 2 et une deuxième face 18 tournée vers l'extérieur. Dans la position de fermeture de la poche, après retournement de la feuille 7, comme représenté sur la figure 4, la deuxième face 18 de la feuille 7 est tournée vers l'intérieur de la poche et la première face 17 vers l'extérieur.

On peut alors retirer la bande de protection 14 et coller le bord de la deuxième face 18, par exemple sur la face 3 de la feuille 2, en scellant ainsi de manière hermétique la poche formée sur le dos de la couche-culotte 1.

Dans cette position de fermeture, l'élément absorbant 5 est enfermé dans la poche ainsi que les liquides et déchets corporels qu'il contient. Ceux-ci ne sont plus capables de dégager à l'extérieur de la poche des odeurs désagréables.

Il est bien entendu que la présente invention n'est pas limitée à la forme de réalisation qui vient d'être décrite et que bien des modifications peuvent y être apportées sans sortir du cadre du présent brevet.

D'une manière avantageuse, la poche peut contenir en outre une serviette de toilette 23, éventuellement imprégnée d'un produit humidifiant ou de nettoyage. Cette serviette peut être utile en cas d'impossibilité de recourir à une salle d'eau au moment du changement de couche.

D'autres dispositions de la feuille étanche aux odeurs et de l'ouverture de poche peuvent aussi être prévues sur le dos de la couche-culotte 1.

## Revendications

1. Bande à jeter (1) du type lange, couche, couche-culotte ou article analogue, comprenant
   un élément absorbant (5),
   une feuille imperméable aux fluides (2)
   dont une première face (4) supporte l'élément absorbant (5),

une enveloppe en forme de poche dans laquelle l'élément absorbant peut être enfermé après usage, et

des moyens de fermeture de l'enveloppe, caractérisée en ce qu'elle comprend en outre une feuille étanche aux odeurs (7) qui est disposée à plat sur une deuxième face (3), opposée à la première (4), de la feuille imperméable aux fluides (2) et qui comporte un bord périphérique (8) dont une partie (9, 10) est fixée sur ladite deuxième face (3), le bord périphérique restant étant libre, en ce que ces deux feuilles forment ensemble ladite enveloppe en forme de poche avec une ouverture de poche ( 12) entre le bord périphérique resté libre (11) de la feuille étanche aux odeurs (7) et la feuille imperméable aux fluides (2), et en ce que lesdits moyens de fermeture (13) sont capables de sceller l'ouverture de poche (12) d'une manière étanche aux odeurs.

2. Bande à jeter suivant la revendication 1, caractérisée en ce que, en position d'utilisation de la bande, la feuille étanche aux odeurs (7) présente une première face (17) adjacente à la feuille imperméable aux fluides (2) et une deuxième face (18), opposée à la première et tournée vers l'extérieur de la bande, et en ce que, en position de fermeture de l'enveloppe (20), la feuille étanche aux odeurs (7) présente au moins partiellement sa deuxième face (18) tournée vers l'intérieur de l'enveloppe (20) et sa première face (17) tournée vers l'extérieur de l'enveloppe.

3. Bande à jeter suivant l'une ou l'autre des revendications 1 et 2, caractérisée en ce que l'ouverture de poche (12) a une dimension suffisante pour permettre l'introduction d'une main à l'intérieur de la poche (20) et un retournement de la feuille étanche aux odeurs (7) autour de l'élément absorbant (5) sans que la main soit souillée par l'élément absorbant contenant des résidus organiques.

4. Bande à jeter suivant l'une quelconque des revendications 1 à 3, caractérisée en ce que la feuille étanche aux odeurs (7) est en une matière imperméable aux fluides, en particulier dans la même matière que ladite feuille imperméable aux fluides (2).

5. Bande à jeter suivant l'une quelconque des revendications 1 à 4, caractérisée en ce que les moyens de fermeture de l'enveloppe (19, 20) consistent en un ruban adhésif (13) double face, au moins partiellement protégé par une languette de protection (14) détachable pendant le stockage et l'usage de la bande à jeter.

6. Bande à jeter suivant l'une des revendications 1 à 5, caractérisée en ce que l'enveloppe en forme de poche (20) contient une serviette de toilette (23), éventuellement imprégnée d'un produit humidifiant ou de nettoyage.

Fig.1

**Fig.2**

**Fig.3**

*Fig.4*

EP 0 496 716 A1

Office européen
des brevets

**RAPPORT DE RECHERCHE EUROPEENNE**

Numero de la demande

EP 92 87 0009

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-4 692 162 (BINKER & MIRANDA) <br> * colonne 3, ligne 14 - ligne 58; figures 1-5 * <br> --- | 1,2,4,5 | A61F13/15 |
| X | US-A-4 857 066 (ALLISON) <br> * colonne 4, ligne 54 - colonne 5, ligne 20; figures 4-6 * <br> --- | 1,2,4,5 | |
| A | US-A-4 923 455 (DEAN & BARABINO) <br> * colonne 5, ligne 7 - ligne 37; figure 1 * | 1,2,4,5 | |
| A | --- | 3-6 | |
| A | US-A-4 182 336 (BLACK) <br> * figures 1-6 * <br> --- | 1,2,4,5 | |
| A | US-A-4 605 403 (TUCKER) <br> * colonne 3, ligne 18 - ligne 24 * <br> --- | 1 | |
| A | DE-U-8 713 699 (HAAS) <br> ----- | 3 | |
| | | | **DOMAINES TECHNIQUES RECHERCHES (Int. Cl.5)** <br> A61F <br> E01H |

Le présent rapport a été établi pour toutes les revendications

| Lieu de la recherche <br> LA HAYE | Date d'achèvement de la recherche <br> 27 MARS 1992 | Examinateur <br> VLECK J. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.........................................................................
& : membre de la même famille, document correspondant

EPO FORM 1503 03.82 (P0402)

8